# EUROPEAN PATENT SPECIFICATION

(11) **EP 3 494 979 B1**
(45) Date of publication and mention of the grant of the patent: **09.07.2025**
(21) Application number: 17837077.1
(22) Date of filing: 03.08.2017
(51) Int. Cl.: A61K 35/28, A61K 35/545, A61P 11/00, A61P 9/00

(54) **ALLEVIATION AND TREATMENT OF ISCHEMIA REPERFUSION-INDUCED LUNG INJURY USING PLURIPOTENT STEM CELLS**
LINDERUNG UND BEHANDLUNG VON ISCHÄMISCHEN REPERFUSIONSINDUZIERTEN LUNGENLÄSIONEN UNTER VERWENDUNG VON PLURIPOTENTEN STAMMZELLEN
SOULAGEMENT ET TRAITEMENT D'UNE LÉSION PULMONAIRE PROVOQUÉE PAR UNE REPERFUSION ISCHÉMIQUE À L'AIDE DE CELLULES SOUCHES PLURIPOTENTES

(30) Priority: 03.08.2016 JP 2016153264
(43) Date of publication of application: 12.06.2019
(73) Proprietor: Yabuki, Hiroshi, Sendai-shi, Miyagi 980-8577 (JP); Okada, Yoshinori, Sendai-shi, Miyagi 980-8577 (JP); Dezawa, Mari, Sendai-shi, Miyagi 980-8577 (JP)
(72) Inventor: Yabuki, Hiroshi, Sendai-shi, Miyagi 980-8577 (JP); Okada, Yoshinori, Sendai-shi, Miyagi 980-8577 (JP); Dezawa, Mari, Sendai-shi, Miyagi 980-8577 (JP)
(74) Representative: AWA Sweden AB
(86) International application number: PCT/JP2017/028328
(87) International publication number: WO 2018/025976

(56) References cited:
- EP-A1- 2 886 123
- EP-B1- 2 683 389
- WO-A2-2008/036374
- JP-A- 2015 159 895
- JING LI ET AL.: "Bone marrow-derived mesenchymal stem cells enhance autophagy via PI3K/AKT signalling to reduce the severity of ischaemia/reperfusion-induced lung injury", JOURNAL OF CELLULAR AND MOLECULAR MEDICINE, vol. 19, no. 10, 2015, pages 2341 - 2351, XP055460266

## Description

### FIELD

The present invention relates to pluripotent stem cells in regenerative medicine. More specifically, it relates to pluripotent stem cells for use in alleviation and/or treatment of ischemia reperfusion-induced lung injury.

### BACKGROUND

In general, tissue ischemia is caused by oxygen deficiency in tissue and induction of inflammation-inducing cytokines, in particular, TNF-α, IL-1β, IL-6 and IL-10, and it yields several kinds of chemical events which have a potential of causing cell dysfunction and necrosis. Once the blood flow is recovered (reperfusion), other series of events yielding further injuries, which occur mostly by forming of free radicals and are considered to be caused by neutrophils activated in a reperfusion site, occur. In most cases, the reperfusion injury is more dangerous than ischemic injury if the ischemia occurs within a short time, in particular.

Ischemia reperfusion-induced injury in lung may possibly occur whenever there is stoppage of blood flow followed by subsequent recovery. As for the time point at which ischemia reperfusion-induced lung injury occurs, reinitiation of blood flow after having organ transplantation, after having artificial cardiopulmonary circulation during heart surgery, after having thoracic surgery of lung cancer or the like accompanying forming of blood vessels or blocking of blood flow, after dissolving blood clots or removing blood clots in pulmonary thromboembolism, or the like, can be mentioned. Currently, for a treatment of ischemia reperfusion-induced injury, administration of adrenocortical steroids or prostacyclin, inhalation of nitrogen monoxide (NO2), improvement of lung preservation solution, and the like are carried out. However, ischemia reperfusion-induced lung injury occurs in 15 to 30% or so of lung transplantation and is related with a rejection in acute phase after lung transplantation and subsequent death (Non Patent Literatures 1 and 2).

In recent years, studies based on cell therapy using stem cells are carried out on various diseases and also a clinical application is made in the field of regenerative medicine. For example, the bone marrow mesenchymal cellular fraction (MSC) is isolated from an adult and is known to have an ability of differentiating into bone, cartilage, adipose cells, nerve cells, skeletal muscle, or the like (Non Patent Literatures 3 and 4). However, MSC is a group of cells in which various kinds of cells are included and the differentiation ability is not entirely clear, and thus there is a huge variation in therapeutic effect. Furthermore, although iPS cells as pluripotent stem cells derived from an adult have been reported (Patent Literature 1), since iPS cells have a high tumor forming ability in addition to that very complex operations like introducing a specific gene to dermal fibroblast cells as mesenchymal cells or a specific compound to somatic cells is required for establishment of iPS cells, a very high hurdle is present in terms of the clinical application. EP 2886123A1 discloses a medical application for use in regenerative medicine that uses pluripotent stem cells (Muse cells). A cell preparation for treating myocardial infarction, and particularly serious massive myocardial infarction and heart failure associated therewith, is disclosed; the cell preparation contains pluripotent stem cells positive for SSEA-3 isolated from biological mesenchymal tissue or cultured mesenchymal cells. WO 2008/036374A2 discloses methods, cells, and compositions of matter for performing stem cell transplants in patients that have not been previously immunosuppressed, specifically methods of matching, methods of treating the stem cell graft, and use of engraftment-assisting cells and agents. EP 2683389B1 discloses compositions comprising mesenchymal stem cell (MSC) derived exosomes, and methods of their use in subjects having certain lung diseases including inflammatory lung disease.

Research by Prof. Dezawa, one of the inventors, has demonstrated that the pluripotency of the mesenchymal cells fraction is exhibited by pluripotent stem cells (Multilineage-differentiating Stress Enduring cells, or Muse cells) that express SSEA-3 (Stage-Specific Embryonic Antigen-3) as a surface antigen, which are present in mesenchymal cell fraction and can be obtained without operation of induction and that this holds potential for application in treatment of diseases bytissue regeneration. It has also been found that Muse cells can be enriched by treating the mesenchymal cells fraction with one or more of different types of stress treatments (Patent Literature 2; Non Patent Literature 5). However, it has not yet been demonstrated that the expected therapeutic effect can be obtained using Muse cells for amelioration and/or treatment of ischemia reperfusion-induced lung injury.

### [CITATION LIST]

### [PATENT LITERATURE]

[PTL 1] JP 4183742 B1
[PTL 2] WO 2011/007900 A

### [NON PATENT LITERATURE]

[NPL 1] Fiser, S.M., et al., J. Heart Lung Transplant., 20, p.631-636 (2001)
[NPL 2] Meyers, B.F., et al., J.Thorac.Cardiovasc.Surg., 120, p.20-26 (2000)
[NPL 3] Dezawa, M., et al., J.Clin.Invest., Vol.113, p.1701-1710 (2004)
[NPL 4] Dezawa, M., et al., Science, Vol.309, p.314-317 (2005)
[NPL 5] Wakao, S, et al., Proc.Natl.Acad.Sci.USA, Vol.108, p.9875-9880 (2011)

### SUMMARY

### [TECHNICAL PROBLEM]

It is an object of the present invention is to provide a novel medical use for pluripotent stem cells (i.e., Muse cells) in regenerative medicine. More specifically, it is an object of the present invention to provide pluripotent stem cells positive for SSEA-3 isolated from mesenchymal tissue of a body or from cultured mesenchymal cells for use in alleviation and/or treatment of ischemia reperfusion-induced lung injury.

### [SOLUTION TO PROBLEM]

The present inventors have found that preparing a rat model of ischemia reperfusion-induced lung injury, and administering Muse cells by pulmonary artery injection, alleviates ischemia reperfusion-induced lung injury, and the present invention has been completed.

Specifically, the present invention provides as follows.
[1] Pluripotent stem cells positive for SSEA-3 isolated from mesenchymal tissue of a body or from cultured mesenchymal cells for use in alleviation and/or treatment of ischemia reperfusion-induced lung injury, wherein the pluripotent stem cells have all of the following properties:
   (i) CD105 positive;
   (ii) low or non-existent telomerase activity;
   (iii) the ability to differentiate into any of the three germ layers;
   (iv) no neoplastic proliferation; and
   (v) self-renewal ability.
[2] Pluripotent stem cells for use according to [1] above, wherein the pluripotent stem cells positive for SSEA-3 have been concentrated by external stress treatment.
[3] Pluripotent stem cells for use according to [1] or [2] above, wherein the pluripotent stem cells are CD105-positive.
[4] Pluripotent stem cells for use according to any one of [1] to [3] above, wherein the pluripotent stem cells are CD117-negative and CD146-negative.
[5] Pluripotent stem cells for use according to any one of [1] to [4] above, wherein the pluripotent stem cells are CD117-negative, CD146-negative, NG2-negative, CD34-negative, vWF-negative, and CD271-negative.
[6] Pluripotent stem cells for use according to any one of [1] to [5] above, wherein the pluripotent stem cells are CD34-negative, CD117-negative, CD146-negative, CD271-negative, NG2-negative, vWF-negative, Sox10-negative, Snai1-negative, Slug-negative, Tyrp1-negative, and Dct negative.
[7] Pluripotent stem cells for use described in any one of [1] to [6] above, wherein the ischemia reperfusion-induced lung injury is lung injury occurring at reinitiation of blood flow after having organ transplantation, after having artificial cardiopulmonary circulation during heart surgery, after having thoracic surgery of lung cancer or the like accompanying forming of blood vessels or blocking of blood flow, and/or after dissolving blood clots or removing blood clots in pulmonary thromboembolism.
[8] Pluripotent stem cells according to any one of [1] to [8] above, wherein the pluripotent stem cells have the ability to engraft into lung tissue.

### [ADVANTAGEOUS EFFECTS OF INVENTION]

According to the present invention, by administering Muse cells to a subject suffering from ischemia reperfusion-induced lung injury, Muse cells are selectively integrated in the disease part of lung tissues and cytokines or tissue protecting factors, or the like are continuously secreted in the tissues by Muse cells, and thus normal lung tissues can be constructed.

### BRIEF DESCRIPTION OF DRAWINGS

FIG. 1 illustrates the result of functional evaluation of lung (P/F ratio) by using Muse cells in rat model of ischemia reperfusion-induced lung injury.
FIG. 2 illustrates the result of functional evaluation of lung (A-aDO₂) by using Muse cells in rat model of ischemia reperfusion-induced lung injury.
FIG. 3 illustrates the result of functional evaluation of lung (left lung compliance) by using Muse cells in rat model of ischemia reperfusion-induced lung injury.
FIG. 4 illustrates the result of histological evaluation of lung (pathological evaluation after hematoxylin · eosin staining) by using Muse cells in rat model of ischemia reperfusion-induced lung injury.
FIG. 5 illustrates the result of histological evaluation of lung (the number of TUNEL positive cells) by using Muse cells in rat model of ischemia reperfusion-induced lung injury.
FIG. 6 illustrates the result of counting the number of human golgi positive cells in lung tissue section of a rat model of ischemia reperfusion-induced lung injury.
FIG. 7A illustrates the expression amount of mRNA of various cytokines in lung tissues of a rat model of ischemia reperfusion-induced lung injury.
FIG. 7B illustrates the expression amount of mRNA of various cytokines in lung tissues of a rat model of ischemia reperfusion-induced lung injury.
FIG. 8 illustrates the result of measuring the proliferation property of alveolar epithelium by using a frozen section of lung tissues from a rat model of ischemia reperfusion-induced lung injury.

### DESCRIPTION OF EMBODIMENTS

The present invention relates to pluripotent stem cells positive for SSEA-3 isolated from mesenchymal tissue of a body or from cultured mesenchymal cells for use in alleviation and/or treatment of ischemia reperfusion-induced lung injury. The present invention will be now explained in greater detail.

### 1. Applicable diseases and their diagnosis

The present invention is directed toward alleviation and treatment of ischemia reperfusion-induced lung injury using a pluripotent stem cells positive for SSEA-3 isolated from mesenchymal tissue of a body or from cultured mesenchymal cells. It is considered that the "ischemia reperfusion-induced lung injury" is a main cause of having functional failure of transplanted lung after lung transplantation, and it is ischemia reperfusion-induced injury occurring in lung after ischemia reperfusion which is also related with chronic rejection happening 3 months after the lung transplantation.

In general, the "ischemia reperfusion-induced injury" indicates an injury which occurs in accordance with generation of various toxic materials in micro circulation within an organ or tissues when blood reperfusion occurs in the organ or tissues in ischemic state. The ischemia · reperfusion theory reported by McCord is the first theory (N.Engl.J.Med., 312, 159-163 (1985)). Degree of the injury varies depending on time and degree of ischemia, type of an organ, or the like. It is also possible that a more severe injury occurs in incomplete ischemia. It is believed that, scratches on vascular endothelial cells and a problem in micro circulation are caused by reperfusion and they are developed into an organ injury. As a mechanism of having the injury, mechanisms like an injury caused by generation of active oxygen such as super oxide (O₂⁻) or hydroxyl radial (HO·) or free radicals such as nitrogen monoxide (NO), an injury caused by generation of various chemical mediators such as various cytokines, endothelin, or arachidonic acid, an injury based on interaction between activated neutrophils and vascular endothelial cells are considered. The injury is caused not only topically but also, secondarily, in major organs of a whole body (remote organ injury). In particular, multiple organ failure is caused by having brain, lung, liver, kidney, or the like as a target organ. The injury is frequently observed after reperfusion therapy for myocardial infarction, brain infarction, mesenteric vascular occlusion, or the like, or after organ transplantation.

The applicable disease to which the pluripotent stem cells of the present invention can be applied is ischemia reperfusion-induced injury, and examples of the disease include, a lung injury occurring at reinitiation of blood flow after having lung transplantation, after having artificial cardiopulmonary circulation during heart surgery, after having thoracic surgery of lung cancer or the like accompanying forming of blood vessels or blocking of blood flow, or after dissolving blood clots or removing blood clots in pulmonary thromboembolism.

According to the present invention, to treat the above applicable disease, by administering (hereinbelow, it may be also generally referred to as "transplant") to a subject the pluripotent stem cells which will be described later, alleviation and/or treatment of the applicable disease can be achieved. As described herein, the "alleviation" means amelioration of various symptoms accompanying the ischemia reperfusion-induced lung injury and suppression of their progress, and it preferably means the amelioration of symptoms to the extent that no significant problem is caused in daily life. Furthermore, the "treatment" indicates suppression or complete removal of various symptoms accompanying the ischemia reperfusion-induced lung injury.

### 2. Cell preparation and pharmaceutical composition

### (1) Pluripotent stem cells

The pluripotent stem cells to be used according to the present invention are typically cells whose existence in the human body was discovered by Prof. Dezawa, one of the present inventors, and which are named "Muse (Multilineage-differentiating Stress Enduring) cells". Muse cells can be obtained from bone marrow fluid and adipose tissue (Ogura, F., et al., Stem Cells Dev., Nov 20, 2013 (Epub) (published on Jan 17, 2014)) or from skin tissue such as dermal connective tissue, and they are widely dispersed throughout the connective tissue of various organs. The cells have the properties of both pluripotent stem cells and mesenchymal stem cells, and are identified as being double-positive for the cell surface markers "SSEA-3 (Stage-specific embryonic antigen-3)" and "CD105". Therefore, Muse cells or cell populations containing Muse cells, for example, can be isolated from body tissue using these antigen markers. Muse cells are also stress-tolerant, and can be concentrated from mesenchymal tissue or cultured mesenchymal cells by different types of stress treatments. A cell fraction with Muse cells enriched by stress treatment may be used as the cell preparation of the present invention. The methods of separation and identification of Muse cells, and their features, are disclosed in detail in International Patent Publication No. WO2011/007900. Also, as reported by Wakao et al. (2011, ibid.), when mesenchymal cells are cultured from the bone marrow or skin and used as a parent population of Muse cells, all of the SSEA-3 positive cells are also CD105-positive. Consequently, when Muse cells are isolated from mesenchymal tissue of a body or cultured mesenchymal stem cells for the cell preparation and pharmaceutical composition of the present invention, the Muse cells may be used after purification with SSEA-3 alone as the antigen marker. Throughout the present specification, pluripotent stem cells (Muse cells) or a cell population containing Muse cells, isolated from mesenchymal tissue of a body or cultured mesenchymal tissue using SSEA-3 as the antigen marker, and which can be used in a cell preparation and pharmaceutical composition for amelioration and/or treatment of perinatal brain damage, may be referred to simply as "SSEA-3 positive cells". Also throughout the present specification, "non-Muse cells" refers to cells that are present in mesenchymal tissue of a body or cultured mesenchymal tissue, and are the remainder of "SSEA-3 positive cells".

In brief, Muse cells or a cell population containing Muse cells can be isolated from body tissue (for example, mesenchymal tissue) using only antibody for the cell surface marker SSEA-3, or using antibodies for both SSEA-3 and CD105. The term "body" here means "mammalian body". According to the present invention, the "body" does not include a fertilized ovum or an embryo at a developmental stage before the blastocyst stage, but it does include an embryo at the developmental stage from the blastocyst stage onward, including the fetus or blastocyst. The mammal may be a primate such as human or monkey, a rodent such as a mouse, rat, rabbit or guinea pig, or a cat, dog, sheep, pig, cow, horse, donkey, goat or ferret. The Muse cells to be used in the cell preparation and pharmaceutical composition of the present invention are clearly distinguished from embryonic stem cells (ES cells) or iPS cells based on separation from body tissue using a direct marker. The term "mesenchymal tissue" refers to tissue from the bone, synovial membrane, fat, blood, bone marrow, skeletal muscle, dermis, ligament, tendon, dental pulp, umbilical cord or umbilical cord blood, or tissues present in various organs. For example, the Muse cells may be obtained from the bone marrow or skin or adipose tissue. Preferably, mesenchymal tissue of a body is harvested and the Muse cells are isolated from the tissue and used. The separating means mentioned above may be used to separate Muse cells from cultured mesenchymal cells such as fibroblasts or bone marrow-derived MSCs. The Muse cells to be used for the cell preparation and pharmaceutical composition of the present invention may be either autologous or allogenic with respect to the recipient.

As mentioned above, Muse cells or a cell population containing Muse cells can be isolated from body tissue using SSEA-3 positivity, or double positivity for SSEA-3 and CD105, as indicators, but human adult skin is known to include various types of stem cells and progenitor cells. However, Muse cells are not identical to these cells. Such stem cells and progenitor cells include skin-derived precursors (SKP), neural crest stem cells (NCSC), melanoblasts (MB), perivascular cells (PC), endothelial precursor cells (EP) and adipose-derived stem cells (ADSC). Muse cells can be separated out as being "non-expressing" for the markers unique to these cells. More specifically, Muse cells can be separated by using non-expression for at least one, and for example, 2, 3, 4, 5, 6, 7, 8, 9, 10 or 11 among 11 markers selected from the group consisting of CD34 (EP and ADSC marker), CD117 (c-kit) (MB marker), CD146 (PC and ADSC marker), CD271 (NGFR) (NCSC marker), NG2 (PC marker), vWF factor (von Willebrand factor) (EP marker), Sox10 (NCSC marker), Snai1 (SKP marker), Slug (SKP marker), Tyrp1 (MB marker) and Dct (MB marker). As an example, non-expression of CD117 and CD146 may be used as the indicator for separation, non-expression of CD117, CD146, NG2, CD34, vWF and CD271 may be used as the indicator, or non-expression of all of the aforementioned 11 markers may be used as the indicator for separation.

The pluripotent stem cells having the aforementioned features to be used for the present invention have all the following properties:
(i) CD105 positive;
(ii) low or non-existent telomerase activity;
(iii) having the ability to differentiate into any of the three germ layers;
(iv) exhibiting no neoplastic proliferation; and
(v) having self-renewal ability.
The pluripotent stem cells of the present invention have all of these properties. As regards (ii), "low or non-existent telomerase activity", this refers to low or non-detectable telomerase activity when using a TRAPEZE XL telomerase detection kit (Millipore), for example. "Low" telomerase activity is, for example, either telomerase activity on the same level as human fibroblasts, which are somatic cells, or telomerase activity of 1/5 and preferably no greater than 1/10 of that of Hela cells. In regard to (iii), the pluripotent stem cells have the ability to differentiate into the three germ layers (endoderm, mesoderm and ectoderm) *in vitro* and *in vivo,* and by induction culturing *in vitro,* for example, they can differentiate into hepatocytes, neurons, skeletal muscle cells, smooth muscle cells, osteocytes or adipocytes. They may also exhibit the ability to differentiate into the three germ layers in the case of transplanting *in vivo* into the testes. They also have the ability to migrate and engraft onto damaged organs (heart, skin, spine, liver, muscle, etc.), by administration into the body via intravenous injection, and differentiate into specific cells of the corresponding tissue. In regard to (iv), the pluripotent stem cells have the property of proliferating at a rate of about every 1.3 days in suspension culture, and growing in suspension culture from a single cell to form an embryoid-like cell mass, slow down the growth at about 14 days; however, when the embryoid-like cell mass is carried into adhesion culture, cell growth resumes and the proliferated cells spread out from the cell mass. They also have the property of not generating teratomas at least for 6 months after transplantation into the testes. In regard to (v), pluripotent stem cells have self-renewal (auto-replicating) ability. The term "self-renewal" means that cells in the embryoid-like cell mass obtained by culturing a single cell in suspension culture can be confirmed to differentiate into cells of all 3 germ layers, and also that when a single cell from the embryoid-like cell mass is again carried into a suspension culture, it forms a next generation embryoid-like cell mass, and reproduce differentiation into three germ layers as well as embryoid-like cell mass in the suspension culture can be confirmed. Self-renewal may be observed once or as several repeated cycles.

The external stress may be any one or a combination of: protease treatment, culturing in a low oxygen concentration, culturing under low-phosphate conditions, culturing with low serum concentration, culturing under low nutritive conditions, culturing under exposure to heat shock, culturing at low temperature, freezing treatment, culturing in the presence of a hazardous substance, culturing in the presence of active oxygen, culturing under mechanical stimulation, culturing with agitating treatment, culturing with pressure treatment, or physical impact. For example, the treatment time with a protease is preferably a total of 0.5 to 36 hours to apply external stress to the cells. The protease concentration may be the concentration used when the cells adhering to a culture vessel are detached, when the cell mass is dispersed into individual cells, or when individual cells are recovered from tissue. The protease is preferably a serine protease, aspartic acid protease, cysteine protease, metalloprotease, glutamic acid protease or N-terminal threonine protease. The protease is also preferably trypsin, collagenase or dispase.

Furthermore, it is believed that pluripotent stem cells having the above characteristics, which are used for the present invention, can engraft in lung tissues having ischemia reperfusion-induced lung injury after being administered to a living body by intravascular injection or the like, and thereafter, as the pluripotent stem cells exhibit an anti-inflammatory activity or a tissue protecting activity and differentiate at the same time to cells constituting the corresponding tissues, ischemia reperfusion-induced lung injury is alleviated and/or treated by them.

### (2) Preparation and use of cell preparation and pharmaceutical composition

A cell preparation and pharmaceutical composition comprising the pluripotent stem cells of the present invention may be obtained by suspending the Muse cells or a cell population containing Muse cells obtained by (1) above, in physiological saline or an appropriate buffer (for example, phosphate-buffered physiological saline, HBSS). In this case, when the number of Muse cells isolated from autologous or allogenic tissue is low, the cells may be cultured before administration for growth until the prescribed cell density is obtained. As already reported (International Patent Publication No. WO2011/007900), Muse cells do not undergo neoplastic transformation, and therefore even if the cells recovered from body tissue are in undifferentiated form, they have low tumorigenicity and are safe. There are no particular restrictions on culturing of the recovered Muse cells, and it may be carried out in ordinary growth medium (for example, α-Minimal Essential Medium (α-MEM) containing 10% newborn calf serum). More specifically, referring to International Patent Publication No. WO2011/007900, suitable medium and additives (for example, antibiotics and serum) may be selected for culturing and growth of the Muse cells, and a solution containing the prescribed density of Muse cells may be prepared. When a cell preparation or pharmaceutical composition of the present invention is to be administered to a human patient, roughly several milliliters of bone marrow fluid may be harvested from human iliac bone, and for example, the bone marrow-derived MSCs may be cultured as adherent cells from the bone marrow fluid to increase them to a number of cells allowing separation of an effective therapeutic amount of Muse cells, after which the Muse cells may be separated out with SSEA-3 antigen marker as the indicator, and autologous or allogenic Muse cells prepared as a cell preparation. As an alternative example, Muse cells that have been separated using SSEA-3 antigen marker as the indicator, and the cells cultured to increase them to an effective therapeutic amount, may then be prepared as a cell preparation of autologous or allogenic Muse cells.

For use of the Muse cells in a cell preparation or pharmaceutical composition, dimethyl sulfoxide (DMSO) or serum albumin may be added to the cell preparation or pharmaceutical composition to protect the cells, and an antibiotic or the like may be added to prevent infiltration and growth of bacteria. In addition, other pharmaceutically acceptable components (for example, carriers, excipients, disintegrators, buffering agents, emulsifying agents, suspending agents, soothing agents, stabilizers, preservatives, antiseptic agents, physiological saline and the like), or cells or components other than Muse cells that are present among MSCs, may be added to the cell preparation or pharmaceutical composition. A person skilled in the art may add such factors and chemical agents to the cell preparation and pharmaceutical composition in appropriate concentrations.

The number of Muse cells to be contained in the cell preparation and pharmaceutical composition prepared above can be suitably adjusted in consideration of sex, age, body weight of a subject, state of lesion, state of cells to be used, or the like such that the effect desired by alleviation and/or treatment of ischemia reperfusion-induced lung injury (e.g., having P/F ratio, A-aDO2, lung compliance, and respiration index in normal state) can be obtained. In Examples 3 to 8 that will be described later, various effects of transplanting Muse cells were examined by using a rat model of ischemia reperfusion-induced lung injury. For the rat model with body weight of about 290 to 340 g, a very excellent effect was obtained by administering SSEA3-positive cells at 1×10⁵ cells/200 µl of PBS (per single animal). From this result, it is expected that an excellent effect is obtained by administering a body weight-based cell amount of about 1×10⁵ cells/kg to about 1×10⁶ cells/kg (e.g., 3×10⁵ cells/kg) to a single individual of mammals (including human). Meanwhile, in order to prevent clogging caused by injection of cells to blood vessel, as a single dosage, it is preferable to contain SSEA-3-positive cells at 1×10⁸ cells/individual or less, preferably 5×10⁷ cells/individual or less, and more preferably 1.8×10⁷ cells/individual or less in the cell preparation. Herein, the individual includes a rat and a human. Furthermore, it is also possible that, till to have a desired therapeutic effect, the cell preparation and pharmaceutical composition of the present invention are administered several times (e.g., 2 to 10 times) with a suitable interval (e.g., twice a day, once a day, twice a week, once a week, or once in 2 weeks). Thus, even though it also depends on the state of a subject, the therapeutically effective amount is preferably an administration amount of about 1×10⁴ cells to about 1×10⁸ cells per single individual in 1 to 10 administrations, for example. The total administration amount for single individual can be, 1×10⁴ cells to 1×10⁹ cells, 1×10⁴ cells to 5×10⁸ cells, 1×10⁴ cells to 1×10⁸ cells, 1×10⁴ cells to 5×10⁷ cells, 1×10⁴ cells to 1×10⁷ cells, 1×10⁴ cells to 5×10⁶ cells, 1×10⁴ cells to 1×10⁶ cells, 1×10⁴ cells to 5×10⁵ cells, 1×10⁴ cells to 1×10⁵ cells, 1×10⁵ cells to 1×10⁹ cells, 1×10⁵ cells to 5×10⁸ cells, 1×10⁵ cells to 1×10⁸ cells, 1×10cells to 5×10⁷ cells, 1×10⁵ cells to 1×10⁷ cells, 1×10cells to 5×10⁶ cells, 1×10⁵ cells to 1×10⁶ cells, 1×10⁶ cells to 1×10⁹ cells, 1×10⁶ cells to 5×10⁸ cells, 1×10⁶ cells to 1×10⁸ cells, 1×10⁶ cells to 5×10⁷ cells, 1×10⁶ cells to 1×10⁷ cells, 1×10⁷ cells to 1×10⁹ cells, 1×10⁷ cells to 5×10cells, 1×10⁷ cells to 1×10⁸ cells, or the like.

Although the pluripotent stem cells of the present invention have, as a subject for the alleviation and treatment, ischemia reperfusion-induced lung injury, for example, lung injury occurring at reinitiation of blood flow after having organ transplantation, after having artificial cardiopulmonary circulation during heart surgery, after having thoracic surgery of lung cancer or the like accompanying forming of blood vessels or blocking of blood flow, or after dissolving blood clots or removing blood clots in pulmonary thromboembolism, the time for administration is after having diagnosis of ischemia reperfusion-induced lung injury according to pulmonary and internal medicinal findings, thoracic ultrasound image diagnosis, MRI or the like after ischemia reperfusion, and it can be also within several months immediately after the diagnosis. For example, the pluripotent stem cells are preferably administered immediately after receiving the injury, but, even at later time after receiving the injury, e.g., 1 hour, 1 day, 1 week, 1 month, 3 months, or 6 months after receiving the injury, it is expected to have the effect of the pluripotent stem cells of the present invention. Furthermore, as it has been confirmed according to the experiment by the inventors of the present invention that Muse cells to be used do not cause an immune response even in a case in which they have a heterologous origin, Muse cells can be suitably administered till to have the effect that is desired for alleviation and treatment of ischemia reperfusion-induced lung injury.

### 3. Preparation of rat model of ischemia reperfusion-induced lung injury

In the present specification, in order to determine the effect of alleviating and treating ischemia reperfusion-induced lung injury by the cell preparation of the present invention, a rat model of ischemia reperfusion-induced lung injury can be prepared and used. As for the rat to be used as a model, Sprague Dawley (SD) strain rat and Wistar/ST strain rat can be generally mentioned, although it is not limited thereto. The method for preparing a rat model of ischemia reperfusion-induced lung injury is well known, and a rat model of ischemia reperfusion-induced lung injury can be prepared according to the method of Manning, E. et al., (Hum.Gene Ther., 21, p.713-727 (2010)). Furthermore, according to an evaluation of pulmonary function to be described later, it can be determined whether a rat model, which has been prepared by the above method, has ischemia reperfusion-induced lung injury.

Muse cells that are used for the present invention have a property of integrating in disease part. Accordingly, with regard to administration of cells, the administration site (e.g., intraperitoneal, intramuscular, disease part), type of blood vessels for their administration (vein and artery), and the like are not limited. Furthermore, as a method for confirming the engraftment of administered Muse cell after their arrival to a disease part, by preparing in advance Muse cell having incorporated gene to express a fluorescent protein (e.g., green fluorescent protein (GFP)) and, after administering to a living body, by observing with a system capable of detecting fluorescence, behavior of Muse cells can be determined. Furthermore, since Muse cells used for the present invention are derived from human, they are in heterogeneous relationship with rat. In an experiment in which heterogeneous cells or the like are administered to a model animal, to suppress the rejection response to heterogeneous cells in a living body, an immunosuppressive agent (cyclosporin or the like) can be administered before or simultaneously with the administration of heterogeneous cells.

### 4. Effect of alleviation and treatment by Muse cells in rat model of ischemia reperfusion-induced lung injury

In an embodiment of the present invention, the cells of the present invention can alleviate and/or treat ischemia reperfusion-induced lung injury and various accompanying symptoms in mammals including human. According to the present invention, by using the rat model of ischemia reperfusion-induced lung injury prepared in the above and experimentally determining the alleviation or the like of symptoms by Muse cells, effect of the Muse cells can be evaluated. The evaluation method can be performed by using a general measurement system for evaluating pulmonary function using rat.

### (1) Functional evaluation

According to the present invention, functional evaluation of lung can be carried out by a general means accepted by a person who is skilled in the pertinent art, and it can be carried out by measuring P/F ratio, A-aDO₂ (alveolo-arterial difference of oxygen partial pressure), and lung compliance, for example.

### (a) P/F ratio

P/F ratio is an oxygenation index that is generally used for functional evaluation of lung, and the ratio is calculated as PaO₂ (arterial oxygen partial pressure)/FiO₂ (inspired oxygen partial pressure). In the case of a healthy person, the normal ratio is 400 to 500. When the ratio is lower than those values, it is considered as a low oxygen state. The oxygen partial pressure can be measured by a blood gas analyzer (e.g., GASTAT-navi (Techno Medica, Yokohama, Japan)). As it is illustrated in Example 3 which will be described later, P/F ratio is 500 or so for a normal rat, and it is less than 100 for a rat model of ischemia reperfusion-induced lung injury. According to administration of Muse cells, P/F ratio can be recovered close to the normal value.

### (b) A-aDO₂ (Alveolo-arterial difference of oxygen partial pressure)

A-aDO₂ means a difference between alveolar oxygen partial pressure (PAO₂) and arterial oxygen partial pressure (PaO₂), and, by obtaining A-aDO2, it can be determined whether or not normal gas exchange occurs within alveoli. Although PaO₂ can be measured by collecting arterial blood and performing a measurement by using blood gas analyzer, PAO₂ cannot be directly measured due to the partial pressure of oxygen contained in alveoli. Due to this reason, as for the PAO₂, a theoretical value is obtained by using an equation (PAO2 = (760 - 47) × 0.21 - PaCO₂/0.8). In the case of a healthy person, the normal value is between 5 and 15. When the value is higher than those values, it is considered that the gas exchange is abnormal. As it is illustrated in Example 3 which will be described later, A-aDO2 is 150 mmHg or so for a normal rat, and it is 500 mmHg or more for a rat model of ischemia reperfusion-induced lung injury. According to administration of Muse cells, A-aDO₂ can be recovered close to the normal value.

### (c) Lung compliance

As an index representing flexibility of lung, lung compliance is represented by a reciprocal of a shrinking property (elasticity) of lung. When pressure for stretching lung (ΔP) is plotted on a horizontal axis and volume change of lung (ΔV) is plotted on a vertical axis, a pressure-volume curve is obtained, and slope of the curve (ΔV/ΔP) corresponds to static lung compliance. In the present study, in order to measure and calculate the lung compliance more conveniently, dynamic lung compliance obtained as a tidal volume (ml)/respiratory pressure (cm H2O) is used. Furthermore, because the rats used for the study are on their growth process and have growing lung, to calibrate the size among individual animals, comparison was made for the value which is obtained by dividing the dynamic lung compliance by bodyweight of an animal (kg) (value in ml/cm H2O/Kg). When the lung compliance is high, it can be said that the lung is in easily stretchable state. On the other hand, when the lung compliance is low, it can be said that the lung is in hard state. As it is illustrated in Example 4 which will be described later, the lung compliance is 1 or so for a normal rat, and it is 0.6 for a rat model of ischemia reperfusion-induced lung injury. According to administration of Muse cells, lung compliance can be recovered close to the normal value. Although it is different from the measurement method using a rat, the normal value is 200 ml/cm H₂O in human.

### (2) Histological evaluation

According to the present invention, histological evaluation can be carried out by a general means accepted by a person who is skilled in the pertinent art, and it can be carried out by hematoxylin · eosin (HE) staining or TUNEL staining, for example.

### (a) Hematoxylin · eosin staining

For histological staining, hematoxylin · eosin staining is frequently used, and it is well known to a person who is skilled in the pertinent art. According to the present invention, for histological evaluation of lung, lung tissues are collected, and after the staining, 5-level grading of 0 to 4+ of acute lung injury is carried out based on the level of four pathological items (edema in alveolar cavity, bleeding in alveolar cavity, blood congestion in alveoli · alveolar septum, and infiltration of inflammatory cells) followed by comparison in each animal test group to perform the evaluation. More specifically, for the evaluation, two people carry out 5-level evaluation for each of the pathological items described above in blind manner, i.e., scoring using the followings; score 0: 0% of lesion, score 1: 1 to 25% of lesion, score 2: 26 to 50% of lesion, score 3: 51 to 75% of lesion, and score 4: 76 to 100% of lesion. As it is illustrated in Example 5 which will be described later, compared to control group to which only PBS has been administered, the treatment group administered with Muse cells shows a decreased average score for any of the above pathological items.

### (b) TUNEL staining

According to the present invention, apoptosis in a lung with injury can be evaluated by using TUNEL (TdT-mediated dUTP nick end labeling) method. It is a method in which fragmented DNA in tissue slices, which occurs during the process of apoptosis, is labeled with biotin-labeled nucleotide, and then reacted with HRP-labeled streptavidin for staining. As a reagent for detection, any of FITC, DAB, and Blue Label can be used. After the staining, by counting the number of TUNEL positive cells under a microscope, comparison can be made for each test group. For example, as it is illustrated in Example 6, when the number of TUNEL positive cells in 10 viewing areas is compared under a microscope, the number of TUNEL positive cells caused by apoptosis can be dramatically reduced in the treatment group using Muse cells compared to a control administered with PBS.

The present invention is more specifically described with the following examples.

### EXAMPLES

### Example 1: Preparation of various cells

### (1) Preparation of mesenchymal stem cells

Human mesenchymal stem cells (MSC) purchased from Lonza, Japan were used. According to previous reports, by using DMEM containing low glucose and L-glutamine (Life Technologies, Carlsbad, U.S.A.) to which 10% fetal bovine serum (FBS) (Sigma-Aidrich, St.Louis, U.S.A.) and kanamycin are added, culture was carried out in a 10 cm dish at conditions including 37°C, 5% CO₂. When the cells reach the cell density of 90% confluency, the cells were released by using 0.25% trypsin-EDTA (Life Technologies, Carlsbad, U.S.A.) followed by 1 : 2 passaging. After repeating similarly the culture and passaging, cells at the 7^{th} to 8^{th} passage were used for the experiment.

### (2) Preparation of Muse cells

Human MSCs were cultured and passaged, and cells at the 7^{th} to 8^{th} passage were used. First, 5 ml of 5% bovine serum albumin (Sigma-Aidrich, St.Louis, U.S.A.) and 1 ml of 100 mM EDTA were added to 44 ml of FluoroBrite DMEM (Life Technologies, Carlsbad, U.S.A.) to prepare a buffer. By using rat IgM anti Stage-Specific Embryonic Antigen-3 (SSEA-3) antibody (1 : 1000, BioLegend, San Diego, U.S.A.) as a primary antibody, the reaction was carried out for 1 hour on ice in buffer prepared with MSC. After that, centrifuge was carried out for 5 minutes at 400 g, and the supernatant was removed and added with 900 µl of buffer followed by washing with gentle pipetting. After repeating this washing operation 3 times, the reaction was carried out for 1 hour on ice by using FITC-conjugated anti rat IgM antibody (1 : 100, Jackson Immunoresearch, Baltimore, U.S.A.) as a secondary antibody. After the reaction with a secondary antibody, the aforementioned washing was repeated 3 times. Subsequently, anti FITC micro beads (1 : 10, Miltenyibiotec, Germany) were allowed to react for 15 minutes on ice, and after washing 2 times, SSEA-3-positive cells were isolated as Muse cells by using MACS (magnetic-activated cell sorting). After separating the cells, part of the cells isolated by MACS were analyzed by using BD FACS Aria (BD Biosciences, Franklin Lakes, U.S.A.) and ratio of the number of SSEA-3-positive cells contained in the isolated cell group was determined. For the experiment, cells showing SSEA-3-positive ratio of 70% or higher according to the analysis by FACS were used. The "Muse cells" that are used for the experiments described below indicate the cells which contain SSEA-3-positive cells at 70% or higher and have been isolated by MACS.

### Example 2: Preparation of rat model of ischemia reperfusion-induced lung injury and administration of various cells

In the present study, four groups in total: PBS group in which PBS is administered to a rat model of ischemia reperfusion-induced lung injury; MSC group in which human MSC is administered thereto; Muse group in which Muse cells are administered thereto; and normal group to which no surgical operation is carried out were prepared. Evaluation of the pulmonary function after ischemia reperfusion-induced lung injury was made with n = 8 for each group. For the PBS group, 200 µl of PBS, for the MSC group, 1.5×10⁵ MSC cells/200 µl of PBS, and for the Muse group, 1.5×10⁵ Muse cells/200 µl of PBS were respectively administered over 1 minute, after piercing left pulmonary artery with 30 G needle immediately after reperfusion.

A male Sprague-Dawley rat with body weight of 290 to 340 g, which is 9 weeks after birth, was used. According to isoflurane inhalation, anesthetization was carried out. When sufficient stabilization is obtained, endobronchial intubation was carried out using 14 G vascular catheter and connected to an artificial respirator for rats. During the anesthetization, the animal was managed to have a tidal volume of 1 ml/100 g, breathing number of 80 times/minute, positive end-expiratory pressure of 2 cm H₂O, and inhaled anesthetic concentration of 1%. The rat was laid on right side, and chest of the rat was opened at the fifth intercostal space by having a cut on the left rear side. Lung tendon was cut off and the left lung was pushed around to sufficiently open the left hilar. After that, 50 units of heparin were administered from left azygos vein. Five minutes after administering the heparin, during the inspiratory end period, left pulmonary artery, left pulmonary vein, and left principal bronchi were blocked with a vascular clip for micro surgery. During the blockage, the lung was covered with a wet gauze and the temperature inside the chest cavity was maintained at 37°C by using a warming mat. Time for the blockage was set at 2 hours according to the report by van der Kaaji NP (Eur J Cardiothorac Surg 2005; 27: 774-782). Immediately after removing the blockage, each of PBS, MSC, and Muse cells was administered through the left pulmonary artery. After that, the lung was closed by suturing the intercostal space, muscular layer, and skin with 3-0 silk thread. After the closure, air ventilation was continued in a state in which inhalation of isoflurane is halted, and after confirming the reinitiation of voluntary breathing, 14 G catheter was removed.

### Example 3: Evaluation of oxygenation property and respiratory function of lung

On Day 3 and Day 5 of the reperfusion, trachea of a rat was cut under systemic anesthetization with isoflurane, and 14 G vascular catheter was inserted to the trachea. Ligation was carried out with 3-0 silk thread and the catheter and trachea were fixed followed by connection between the catheter and an artificial respirator. Chest was opened by median sternotomy. Diaphragms at left and right sides were cut, and, by using a gauze, an overflow of abdominal organs into chest cavity was prevented. After removing the right hilar under a microscope, the right pulmonary artery was ligated. After that, air ventilation was carried out for 5 minutes with inhaling oxygen concentration of 100%, single respiratory volume of 1 ml/100 g of bodyweight, breathing number of 80 times/minute, and positive end-expiratory pressure of 2 cm H₂O, and then blood was collected from ascending aorta. Blood gas analysis was carried out, and arterial oxygen partial pressure/inspired oxygen partial pressure (P/F ratio) and also A-aDO₂ (A-aDO2 = 713 - arterial carbon dioxide partial pressure/0.8 - arterial oxygen partial pressure) calculated from the following equation were compared among each group. As for the blood gas analysis, the measurement was carried out by using GASTAT-navi (Techno Medica, Yokohama, Japan). Evaluation was made with n = 8 for each group.

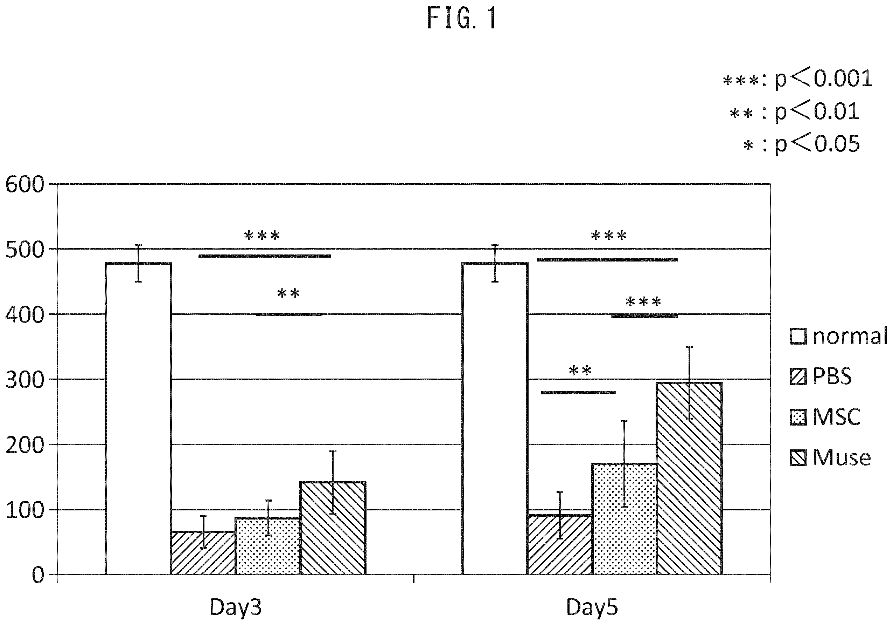

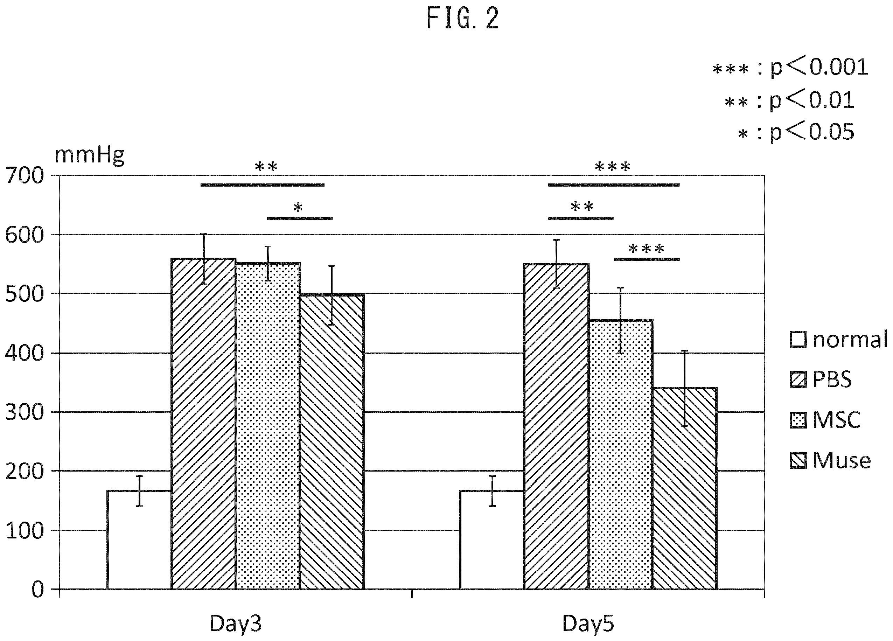

On day 3 from the reperfusion (Day 3) and on day 5 from the reperfusion (Day 5), arterial blood gas analysis was carried out, and P/F ratio and A-aDO₂ were measured and compared. With regard to the P/F ratio, a statistically significant difference was recognized from, on Day 3, the Muse group and the PBS group (p < 0.001), and the Muse group and the MSC group (p < 0.01), and also, on Day 5, from the Muse group and the PBS group (p < 0.001), the Muse group and the MSC group (p < 0.001), and the MSC group and the PBS group (p < 0.01) (Fig. 1). With regard to the A-aDO₂, a statistically significant difference was recognized from, on Day 3, the Muse group and the PBS group (p < 0.01), and the Muse group and the MSC group (p < 0.05), and also, on Day 5, from the Muse group and the PBS group (p < 0.001), the Muse group and the MSC group (p < 0.001), and the MSC group and the PBS group (p < 0.01) (Fig. 2). From the above results, as the pulmonary function in a rat model of ischemia reperfusion-induced lung injury is improved according to the administration of Muse cells, it was able to confirm the therapeutic effectiveness of Muse cells in ischemia reperfusion-induced lung injury.

### Example 4: Evaluation of left lung compliance

After the blood gas analysis, the right hilar was blocked with mosquito pean forceps. The respiration number was set at 80 times/minute and minute respiratory volume was increased gradually from 40 ml to 200 ml, and then the respiratory pressure at each respiratory volume was measured. After adjustment by dividing single respiratory volume by bodyweight of the animal, respiratory volume/respiratory pressure (ml/kg/cm H₂O) was obtained and, as lung compliance, it was compared among each group. Evaluation was made with n = 8 for each group.

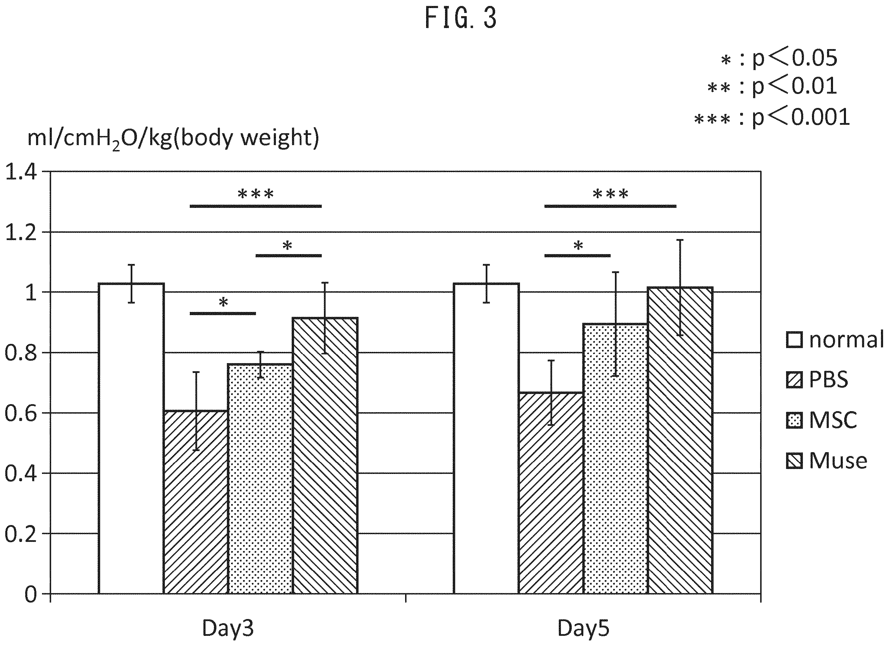

On day 3 from the reperfusion (Day 3) and on day 5 from the reperfusion (Day 5), left lung compliance was measured and compared. A statistically significant difference was recognized from, on Day 3, the Muse group and the PBS group (p < 0.001), the Muse group and the MSC group (p < 0.05), and the MSC group and the PBS group (p < 0.05), and also, on Day 5, from the Muse group and the PBS group (p < 0.001), and the MSC group and the PBS group (p < 0.05) (Fig. 3). Similar to Example 3, it was able to confirm the therapeutic effectiveness of Muse cells in ischemia reperfusion-induced lung injury.

### Example 5: Histological evaluation

After measuring the left lung compliance, a cardiopulmonary block was collected and injected with 4% paraformaldehyde at pressure of 10 cm H₂O from bronchi. The left lung was fixed for 24 hours in 4% paraformaldehyde. After the fixing, the lung tissues were dissected and half of them was embedded in paraffin while the other half was embedded in O.C.T compound, thus preparing each of the paraffin section and frozen section. Thinly sliced sections were prepared such that the paraffin section is 3 µm and the frozen section is 8 µm, respectively.

### (1) Pathological determination of ischemia reperfusion-induced lung injury

By using the paraffin section, hematoxylin · eosin staining was carried out, and 5-level grading of 0 to 4+ of acute lung injury based on the level of four pathological items (edema in alveolar cavity, bleeding in alveolar cavity, blood congestion in alveoli · alveolar septum, and infiltration of inflammatory cells) was carried out on the basis of the report by Okada Y (Transplantation 1997; 64: 801-806), and comparison was made among each group. On Day 3 and Day 5, the evaluation was made with n = 8 for each group.

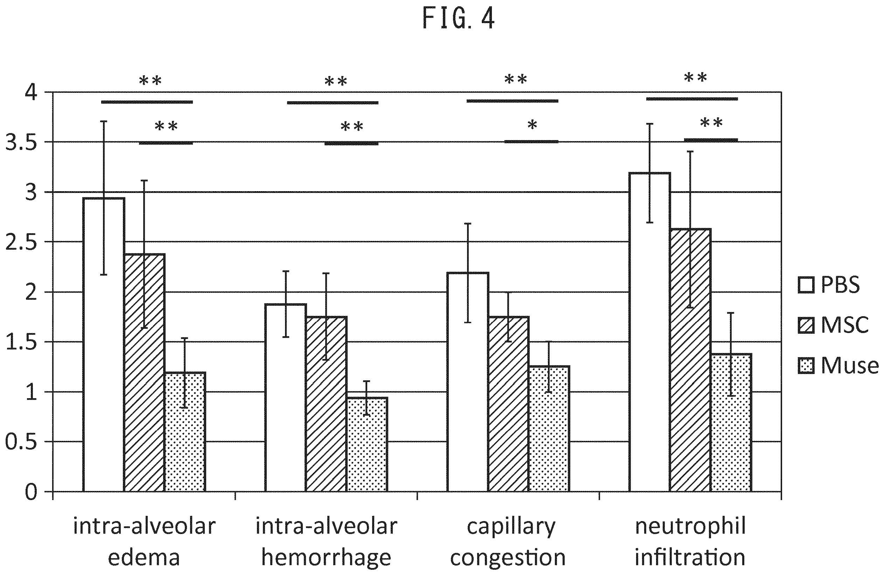

On Day 3 and Day 5, HE staining was carried out, and 5-level evaluation of 0 to 4 was made for the above four pathological items. On Day 3, a statistically significant difference was recognized from the Muse group and the PBS group (p < 0.01), and the Muse group and the MSC group (p < 0.01) with regard to edema in alveoli, the Muse group and the PBS group (p < 0.01), and the Muse group and the MSC group (p < 0.01) with regard to bleeding inside alveoli, while the statistically significant difference is recognized from the Muse group and the PBS group (p < 0.01), and the Muse group and the MSC group (p < 0.05) with regard to blood congestion in peripheral blood vessels, and from the Muse group and the PBS group (p < 0.01), the Muse group and the MSC group (p < 0.01) with regard to neutrophil infiltration (Fig. 4). On Day 5, a statistically significant difference was recognized from the Muse group and the PBS group (p < 0.01), and the Muse group and the MSC group (p < 0.05) with regard to neutrophil infiltration (data not shown). Accordingly, compared to the control group administered with PBS only, since the average score for any of the pathological items is lower in the treatment group administered with Muse cells, it was able to confirm that the Muse cells are effective for a treatment of ischemia reperfusion-induced lung injury.

### (2) Evaluation of apoptosis in injured lung of rat

In order to evaluate apoptosis in injured lung, TUNEL (TdT-mediated dUTP nick end labeling) method was carried out. For TUNEL method, staining was carried out by using DeadEnd (trademark) Fluorometric TUNEL System (Promega, Wisconsin, U.S.A.) according to the protocols enclosed therewith. Observation was made for 10 viewing areas at x200 enlargement, and the total number of TUNEL positive cells in 10 viewing areas was counted and compared among each group. On Day 3 and Day 5, the evaluation was made with n = 4 for each group.

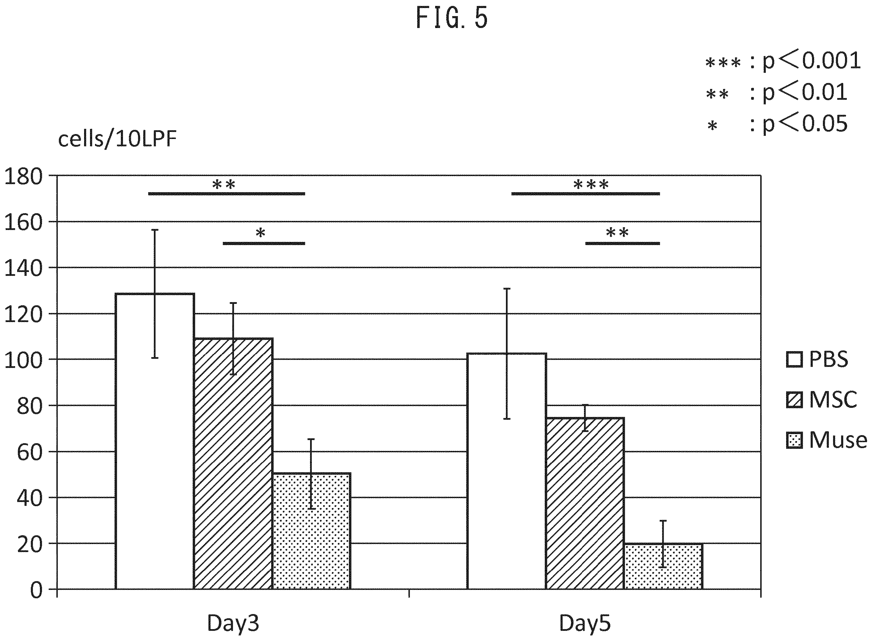

On Day 3 and Day 5, cells with apoptosis were detected by using the left lung section from the PBS group, the MSC group, and the Muse group, and by carrying out TUNEL method. According to the observation of 10 viewing areas at x200 enlargement, the number of TUNEL positive cells was compared (Fig. 5). On Day 3, a decrease in significant sense was observed from the Muse group compared to the PBS group (p < 0.01) and the MSC group (p < 0.05), and, on Day 5, a decrease in significant sense was observed from the Muse group compared to the PBS group (p < 0.001), the MSC group (p < 0.01). From those results, it was found that the number of the cells which eventually have apoptosis as caused by ischemia reperfusion-induced lung injury can be dramatically reduced in the treatment group in which Muse cells are used.

### Example 6: Evaluation of number of human cells in injured lung of rat

By using the frozen section, a reaction with rabbit anti human golgi antibody (1 : 50, Abcam, Cambridge, UK) as a primary antibody was carried out overnight at 4°C. A reaction with Cy3-conjugated donkey anti rabbit IgG antibody (1 : 500, Jackson Immunoresearch, Baltimore, U.S.A.) as a secondary antibody was carried out for 2 hours at room temperature. By performing immunohistochemistry, the number of the anti human golgi antibody positive cells in the MSC group and the Muse group was counted. Three sections were prepared for each animal of both 2 groups. By measuring the area of the section and counting the total number of positive cells for each section, the number of the anti human golgi antibody positive cells per unit area was compared between the 2 groups. On Day 3 and Day 5, the evaluation was made with n = 4 for each group.

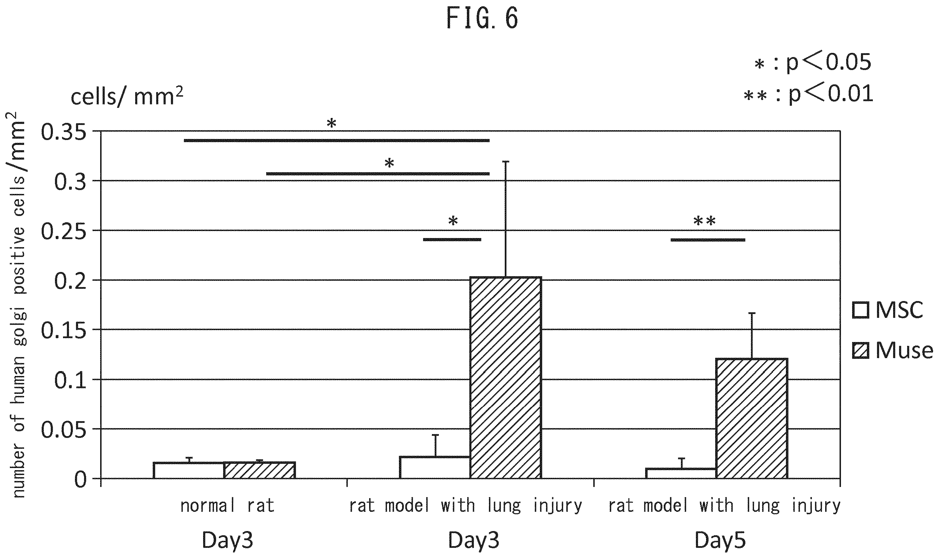

On Day 3 and Day 5, immunohistochemistry using anti human golgi antibody was carried out for left lung tissues, and the number of the human golgi positive cells was counted for each section, three sections for each individual animal. On Day 3, from the group in which Muse cells are administered to a rat model with ischemia reperfusion-induced lung injury, more human cells have remained in significant sense compared to the group in which MSC is administered to a normal rat (p < 0.05), the group in which Muse cells are administered to a normal rat (p < 0.05), and the group in which MSC is administered to a rat model of ischemia reperfusion-induced lung injury (p < 0.05). Furthermore, on Day 5, from the group in which Muse cells are administered to a rat model of ischemia reperfusion-induced lung injury, more human cells have remained in significant sense compared to the group in which MSC is administered to a rat model with ischemia reperfusion-induced lung injury (p < 0.01) (Fig. 6). Namely, it can be said that the Muse cells can more efficiently and continuously act on injured lung than MSC.

### Example 7: Evaluation of protein expressed in injured lung of rat

To compare the protein expressed in injured lung, proteins were extracted from injured lung and subjected to Western blot. On Day 3 after reperfusion, left lung tissues were collected from the PBS group, the MSC group, and the Muse group, and, after homogenization using a biomasher, reacted in Lysis Buffer (20 mM Tris-HCl,150 mM NaCl, 1% Triton X-100) added with a protease inhibitor cocktail (Roche, Mannheim, Germany) for 15 minutes on ice. Amount of the extracted protein was quantified by Bradford method. Subsequently, x2 Sample Buffer (30% glycerol, 4% sodium dodecyl sulfate, 125 mM Tris-HCl (pH 6.8), 100 mM dithiothreitol, 10 mM EDTA, 10% β-mercaptomethanol, and 0.1% bromophenol blue) in the same volume as Lysis Buffer was added thereto followed by incubation for 10 minutes at 100°C. Each sample was electrophoresed, in an amount of 50 µg, by using 10%, 15% SuperSep Ace (Wako, Osaka, Japan), and then transferred onto an Immobilon-P transfer membrane (Merck Millipore, Billerica, U.S.A.). After blocking with 5% skim milk (Nakarai Tesque, Kyoto, Japan) for 1 hour at 4°C, the reaction was carried out overnight at 4°C by using rabbit anti IL-10 antibody (1 : 2500, Abcam, Cambridge, UK), rabbit anti IL-1β antibody (1 : 5000, Merck Millipore Billerica, U.S.A), mouse anti IL-6 antibody (1 : 2000, Abcam, Cambridge, UK), goat anti KGF antibody (1 : 2000, R&D systems, Minneapolis, U.S.A.), rabbit anti PGE2 antibody (1 : 1000, Bioss, Boston, U.S.A.), rabbit anti SDF (Stem cell derived factor)-1 antibody, rabbit anti epithelial sodium channel (ENaC) antibody (1 : 500, Thermo Fisher Scientific, Waltham, U.S.A.), rabbit anti Akt antibody (1 : 2000, Cell Signaling Technology, Danvers, U.S.A.), rabbit anti Bcl-2 antibody (1 : 500 Abcam, Cambridge, UK), or mouse anti β-actin antibody (1 : 10000, Abcam, Cambridge, UK), diluted with 5% skim milk, as a primary antibody. Then, a reaction was carried out for 1 hour at room temperature by using peroxidase-conjugated goat anti mouse IgG antibody (1 : 10000), peroxidase-conjugated goat anti rabbit IgG antibody (1 : 15000), or peroxidase-conjugated donkey anti goat IgG antibody (1 : 5000) as a secondary antibody. For color development, Piece Western Blotting Substrate Plus (Thermo Fisher Scientific, Waltham, U.S.A.) was used and color development was allowed to occur for 5 minutes. Photographic image was taken by using LAS-4000 IR Multi Color (Fuji Film, Tokyo, Japan), and expression level of each protein relative to β-actin was calculated and compared among each group.

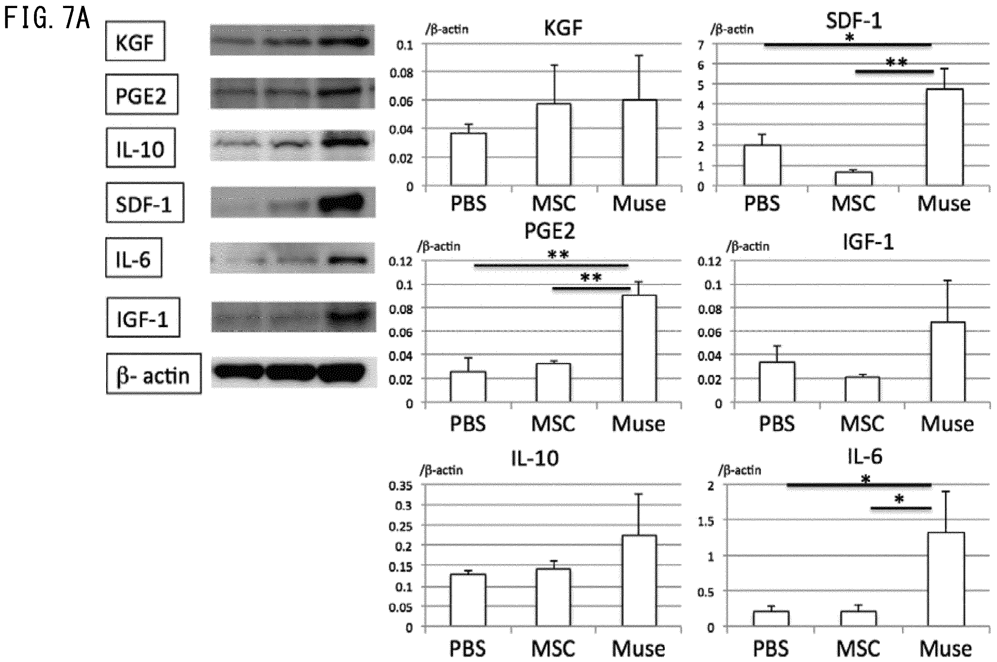

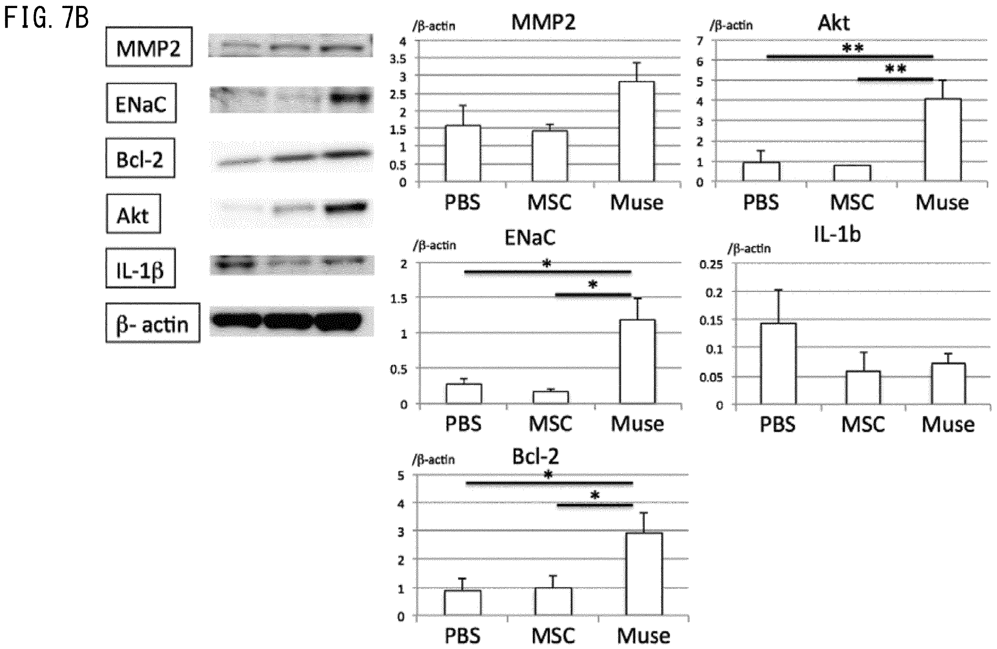

On Day 3, proteins were extracted from left lung tissues of the PBS group, the MSC group, and the Muse group and subjected to Western blot. The result of comparing the band density among each group is shown in Fig. 7. The expression was higher in statistically significant sense in the Muse group and the MSC group, as PGE2 (p < 0.01), SDF-1 (p < 0.05), IL-6 (p < 0.05), ENaC (p < 0.05), Bcl-2 (p < 0.05), and Akt (p < 0.01), and also in the Muse group and the PBS group, PGE2 (p < 0.01), SDF-1 (p < 0.01), IL-6 (p < 0.05), ENaC (p < 0.05), Bcl-2 (p < 0.05), and Akt (p < 0.01). Although a difference in statistical significant sense was not recognized, the expression of KGF, Il-10, MMP2, and IGF-1 was high in the Muse group while the expression of IL-1b was low in the Muse group.

By administering Muse cells to a rat model of ischemia reperfusion-induced lung injury, more excellent function-improving effect was obtained than administration of MSC. According to Western blot of injured lung, KGF, PGE2, IL-10, SDF-1, IL-6, MMP-2, IGF-1, ENaC, Bcl-2, and Akt are expressed at high level while IL-1β is expressed at low level in the Muse group. In sodium ion channels expressed in type I alveolar epithelial cells and type II alveolar epithelial cells, ENaC plays an important role in fluid clearance in alveolar cavity based on absorption of sodium ions, and it is also involved with an occurrence of pulmonary edema in acute lung injury including ischemia reperfusion-induced lung injury. KGF induces the expression of ENaC to augment alveolar fluid clearance. It is considered that, as the expression of ENaC is promoted, alveolar fluid clearance is augmented to contribute to the amelioration of alveolar edema in pathological tissues, and thus being involved with an improvement of the pulmonary function.

IL-6, IGF-1, and SDF-1 achieve tissue restoration by promoting cell proliferation. In the Muse group in which those cytokines are expressed at high level, the number of the cells all in division period is higher in significant sense in the evaluation of proliferation property of type I alveolar epithelial cells and type II alveolar epithelial cells using PCNA. Furthermore, apoptosis is largely involved with an occurrence of pulmonary dysfunction in ischemia reperfusion-induced lung injury, and Bcl-2 and Akt are the proteins which have an activity of suppressing apoptosis during apoptosis pathway. As is evident from the results that the expression of those proteins has increased in the Muse group and the number of the cells that are positive under TUNEL method has decreased in the Muse group, apoptosis is more strongly suppressed in the Muse group. Namely, it can be said that, in the Muse group, there is a large amount of the type I and type II alveolar epithelial cells which survive after ischemia reperfusion-induced injury. Survival or proliferation of a large amount of the type I and type II alveolar epithelial cells means an increase in effective gas exchange area and production of lung surfactant protein, respectively, and it is considered that each of them directly contributes to an improvement of the oxygenation property and lung compliance. Furthermore, the increase in the number of the type I and type II alveolar epithelial cells in injured lung means an increase in ENaC which is expressed in alveolar epithelial cells, and it is considered that this increase also contributes to the improvement of edema in alveoli.

Other than direct activities like immunosuppressive effect and effect of protecting vascular endothelial cells by suppressing the proliferation of T cells, PGE2 has an anti-inflammatory activity through IL-10. In the Muse group in which PGE2 is expressed at high level, IL-10 having a potent anti-inflammatory activity and immunosuppressive activity is also expressed at high level, and it is considered that, in the Muse group, it is involved with a decrease in IL-1b as an inflammatory cytokine and a decrease in infiltration of neutrophils in pathological tissues, and thus contributing to the improvement of pulmonary function.

### Example 8: Evaluation of alveolar epithelium proliferation property in injured lung of rat

After carrying out antigen retrieval by using the frozen section and microwave for 20 minutes at 80°C in Tris-EDTA (pH 9.0), the reaction was carried out overnight at 4°C by using, as a primary antibody, mouse anti PCNA (Proliferating cell nuclear antigen) antibody (1 : 500, Abcam, Cambridge, UK) as a cell marker of proliferation period, rabbit anti Pro Surfactant protein C antibody (1 : 1000, Merck Millipore, Billerica, U.S.A.) as a marker for type 2 alveolar epithelial cells, and rabbit anti Aquaporin 5 antibody (1 : 250, Abcam, Cambridge, UK) as a marker for type 1 alveolar epithelial cells. Then, the reaction was carried out for 2 hours at room temperature by using each of FITC-conjugated goat anti mouse IgG antibody (1 : 500, Jackson Immunoresearch, Baltimore, U.S.A.) and Cy3-conjugated donkey anti rabbit IgG antibody (1 : 500, Jackson Immunoresearch, Baltimore, U.S.A.) as a secondary antibody. Observation of viewing areas at x200 enlargement was made and double positive cells for PCNA and Pro Surfactant protein C, PCNA and Aquaporin 5, respectively, were counted for 10 viewing areas. Each of them was compared, as the number of type 2 alveolar epithelial cells and the number of type 1 alveolar epithelial cells in proliferation period, among the PBS group, the MSC group, and the Muse group. On Day 3 and Day 5 after the reperfusion, the evaluation was made with n = 4 for each group.

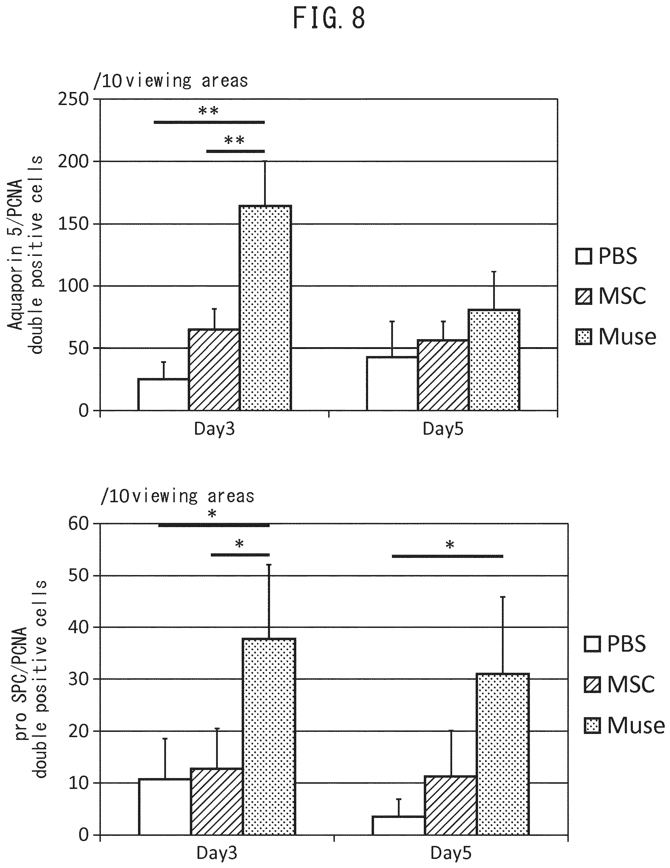

On Day 3 and Day 5, the number of the cells in division period was counted by using anti PCNA antibody. Co-staining was carried out with aquaporin 5 as a type I alveolar epithelial cell marker and pro SPC antibody as a type II alveolar epithelial cell marker, and the number of the double positive cells observed in 10 viewing areas at x200 enlargement was counted. The results are shown in Fig. 8. On Day 3, the number of the double positive cells for PCNA and aquaporin 5 was higher in statistically significant sense in the Muse group compared to the MSC group (p < 0.01) and the PBS group (p < 0.01). On Day 5, the number of the double positive cells was high in the Muse group although a statistically significant difference was not recognized. On Day 3, the number of the double positive cells for PCNA and pro SPC was higher in significant sense in the Muse group compared to the MSC group (p < 0.05) and the PBS group (p < 0.05). On Day 5, the number of the double positive cells was higher in the Muse group compared to other two groups. A statistically significant difference was recognized particularly from the comparison with the PBS group (p < 0.05).

### INDUSTRIAL APPLICABILITY

The pluripotent stem cells of the present invention can be applied for alleviation and treatment of ischemia reperfusion-induced lung injury.

## Claims

1. Pluripotent stem cells positive for SSEA-3 isolated from mesenchymal tissue of a body or from cultured mesenchymal cells for use in alleviation and/or treatment of ischemia reperfusion-induced lung injury, wherein the pluripotent stem cells have all of the following properties:
(i) CD105 positive;
(ii) low or non-existent telomerase activity;
(iii) the ability to differentiate into any of the three germ layers;
(iv) no neoplastic proliferation; and
(v) self-renewal ability.

2. Pluripotent stem cells for use according to claim 1, wherein the pluripotent stem cells positive for SSEA-3 have been concentrated by external stress treatment.

3. Pluripotent stem cells for use according to any one of claims 1 to 2, wherein the pluripotent stem cells are CD117-negative and CD146-negative.

4. Pluripotent stem cells for use according to any one of claims 1 to 3, wherein the pluripotent stem cells are CD117-negative, CD146-negative, NG2-negative, CD34-negative, vWF-negative, and CD271-negative.

5. Pluripotent stem cells for use according to any one of claims 1 to 4, wherein the pluripotent stem cells are CD34-negative, CD117-negative, CD146-negative, CD271-negative, NG2-negative, vWF-negative, Sox10-negative, Snailnegative, Slug-negative, Tyrp1-negative, and Dct negative.

6. Pluripotent stem cells for use according to any one of claims 1 to 5 above, wherein the ischemia reperfusion-induced lung injury is lung injury occurring at reinitiation of blood flow after having organ transplantation, after having artificial cardiopulmonary circulation during heart surgery, after having thoracic surgery of lung cancer or the like accompanying forming of blood vessels or blocking of blood flow, and/or after dissolving blood clots or removing blood clots in pulmonary thromboembolism.

7. Pluripotent stem cells for use according to any one of claims 1 to 6, wherein the pluripotent stem cells have the ability to engraft into lung tissue.

## Patentansprüche

1. SSEA-3-positive pluripotente Stammzellen, die aus mesenchymalem Gewebe eines Körpers oder aus kultivierten mesenchymalen Zellen isoliert sind, zur Verwendung bei der Linderung und/oder Behandlung von ischämischen reperfusionsinduzierten Lungenläsionen, wobei die pluripotenten Stammzellen alle der folgenden Eigenschaften aufweisen:
(i) CD105-positiv
(ii) niedrige oder nicht vorhandene Telomerase-Aktivität;
(iii) die Fähigkeit zur Differenzierung zu einem der drei Keimblätter;
(iv) keine neoplastische Proliferation; und
(v) Selbsterneuerungsfähigkeit.

2. Pluripotente Stammzellen zur Verwendung nach Anspruch 1, wobei die SSEA-3-positiven pluripotenten Stammzellen durch externe Stressbehandlung konzentriert worden sind.

3. Pluripotente Stammzellen zur Verwendung nach einem der Ansprüche 1 bis 2, wobei die pluripotenten Stammzellen CD117-negativ und CD146-negativ sind.

4. Pluripotente Stammzellen zur Verwendung nach einem der Ansprüche 1 bis 3, wobei die pluripotenten Stammzellen CD117-negativ, CD146-negativ, NG2-negativ, CD34-negativ, vWF-negativ und CD271-negativ sind.

5. Pluripotente Stammzellen zur Verwendung nach einem der Ansprüche 1 bis 4, wobei die pluripotenten Stammzellen CD34-negativ, CD117-negativ, CD146-negativ, CD271-negativ, NG2-negativ, vWF-negativ, Sox10-negativ, Snail1-negativ, Slug-negativ, Tyrp1-negativ und Dct-negativ sind.

6. Pluripotente Stammzellen zur Verwendung nach einem der vorhergehenden Ansprüche 1 bis 5, wobei es sich bei den ischämischen reperfusionsinduzierten Lungenläsionen um Lungenläsionen handelt, die bei der Reinitiierung der Durchblutung nach Durchführung einer Organtransplantation, nach Durchführung einer künstlichen kardiopulmonalen Zirkulation während einer Herzoperation, nach Durchführung einer Thoraxoperation bei Lungenkrebs oder dergleichen mit begleitender Bildung von Blutgefäßen oder Blockierung der Durchblutung und/oder nach dem Auflösen von Blutgerinnseln oder Entfernen von Blutgerinnseln bei pulmonaler Thromboembolie stattfinden.

7. Pluripotente Stammzellen zur Verwendung nach einem der Ansprüche 1 bis 6, wobei die pluripotenten Stammzellen die Fähigkeit haben, sich in Lungengewebe einzupflanzen.

## Revendications

1. Cellules souches pluripotentes positives pour SSEA-3 isolées du tissu mésenchymal d'un corps ou de cellules mésenchymales cultivées destinées à être utilisées dans le soulagement et/ou le traitement d'une lésion pulmonaire provoquée par reperfusion ischémique, les cellules souches pluripotentes ayant toutes les propriétés suivantes
(i) positives pour CD105
(ii) activité télomérase faible ou inexistante
(iii) capacité de se différencier en l'une quelconque des trois couches germinales,
(iv) aucune prolifération néoplasique et
(v) capacité à l'auto-renouvellement.

2. Cellules souches pluripotentes destinées à être utilisées selon la revendication 1, dans lesquelles les cellules souches pluripotentes positives pour SSEA-3 ont été concentrées par traitement de stress externe.

3. Cellules souches pluripotentes destinées à être utilisées selon l'une quelconque des revendications 1 à 2, dans lesquelles les cellules souches pluripotentes sont négatives pour CD117 et négatives pour CD146.

4. Cellules souches pluripotentes destinées à être utilisées selon l'une quelconque des revendications 1 à 3, dans lesquelles les cellules souches pluripotentes sont négatives pour CD117, négatives pour CD146, négatives pour NG2, négatives pour CD34, négatives pour vWF et négatives pour CD271.

5. Cellules souches pluripotentes destinées à être utilisées selon l'une quelconque des revendications 1 à 4, dans lesquelles les cellules souches pluripotentes sont négatives pour CD34, négatives pour CD117, négatives pour CD146, négatives pour CD271, négatives pour NG2, négatives pour vWF, négatives pour Sox-10, négatives pour Snai 1, négatives pour Slug, négatives pour Tyrp1 et négatives pour Dct.

6. Cellules souches pluripotentes destinées à être utilisées selon l'une quelconque des revendications 1 à 5 ci-dessus, dans lesquelles la lésion pulmonaire provoquée par reperfusion ischémique est une lésion pulmonaire se produisant à la réinitiation de l'écoulement sanguin après avoir une greffe d'organe, après avoir une circulation cardiopulmonaire artificielle durant une chirurgie cardiaque, après avoir une chirurgie thoracique du cancer du poumon ou similaire accompagnant la formation de vaisseaux sanguins ou le blocage de l'écoulement sanguin et/ou après la dissolution de caillots sanguins ou l'enlèvement de caillots sanguins dans le thromboembolisme pulmonaire.

7. Cellules souches pluripotentes destinées à être utilisées selon l'une quelconque des revendications 1 à 6, dans lesquelles les cellules souches pluripotentes ont la capacité de se greffer dans le tissu pulmonaire.
